# EUROPEAN PATENT SPECIFICATION

(11) **EP 2 030 586 B1**
(45) Date of publication and mention of the grant of the patent: **11.05.2011**
(21) Application number: 07017170.7
(22) Date of filing: 01.09.2007
(51) Int. Cl.: A61B 18/20

(54) **Laser system for medical and cosmetic applications**
Lasersystem für medizinische und kosmetische Anwendungen
Système laser pour applications médicales et cosmétiques

(43) Date of publication of application: 04.03.2009
(73) Proprietor: Fotona d.d., 1210 Ljubljana (SI)
(72) Inventor: Nemes, Karols, 1000 Ljubljana (SI); Lukac, Matjaz, 1000 Ljubljana (SI)
(74) Representative: Riedel, Peter

(56) References cited:
- EP-A- 1 329 773
- WO-A-00/53261
- WO-A-2004/086947
- WO-A-2007/039854
- US-A- 5 885 275
- US-A1- 2005 061 779

## Description

The invention relates to a laser system for medical and cosmetic applications in accordance with the features of the preamble of claim 1.

Lasers are used in medical applications in both ablative and non-ablative procedures. Typically, laser energy is delivered to the treatment site by means of an optical delivery system. In ablative procedures, the laser energy either melts away or breaks the tissue by means of thermally induced microexplosions. The tissue particles that are torn off the bulk tissue are in the process ejected away from the treatment site, and, as a result of this, some of these particles will collect on the surface(s) of external optical elements of the optical delivery system. A typical external optical element consists of a focusing lens through which laser light is transmitted onto the tissue. The pollution of the external optical element with ejected tissue debris may lead to a reduced transmission, and, in the worst case, to complete blockage of the laser light. This has an unpredictable outcome on the safety and efficacy of the laser treatment as it is not known how much laser light is being transmitted to the tissue. An even worse result of debris collecting on the optical element is that the polluted optical element can become irreversibly damaged. Namely, the presence of an absorbing material or impurity on an optical surface significantly reduces the threshold for the laser-caused optical damage. This is particularly critical when using pulsed lasers with high pulse power and/or energy. The damage to the optical element can result in an additionally reduced transmission, undesirable laser light scattering on the damaged surface, or can even cause a complete failure of the optical element.

External optical elements of the laser optical delivery system can even become polluted with ejected debris when treatments are non-ablative. In non-ablative procedures, for example, hair removal, skin rejuvenation, vascular treatments or tattoo removal, the major goal of the procedure is to heat a target, such as a hair follicle or a vessel, inside the tissue without ablating or removing the upper tissue layers. However, even during such non-ablative procedures debris can be ejected from the treatment site. This can occur when, for example, there is an excessively absorbing skin imperfection at the treatment site. Also, during hair removal procedures it happens frequently that hair follicles are being ejected out of the skin as a result of being heated by the laser light. And finally, pollution can result also from the laser light being absorbed in a cooling or pain relieving substance (gel) that is sometimes applied to the treated tissue surface.

A typical solution for the above problem is to use lower cost optical windows to protect sensitive laser optics. Such solution is e. g. known from US 5,885,275, disclosing an energy transmissive optical window, which is in operation pressed onto the target surface, and which protects the optical output element of the handpiece. However, apart from reducing the cost of replacing the optical elements, this solution suffers from the same problems as described above: debris will collect on the optical window, resulting in reduced transmission and possibly optical damage to the window. The optical transmission is varying over time in an uncontrolled manner, resulting in an undefined output level.

The invention has the object to further develop a laser system of the aforementioned kind such that its operational safety is improved.

This object is solved by a laser system having the features of claim 1.

A laser system for medical and cosmetic applications is proposed which comprises an optical system for guiding the laser beam to a target surface. The optical delivery system has an external optical element facing the target surface. At the exit side of the external optical element, a mechanical filter in the form of a protective screen is provided for shielding the external optical element from particles that are ejected by the laser beam. The mechanical filter in the form of a protective screen shields the external surface of the optical element that is facing the target surface from particles that are ejected by the laser beam. The external optical element maintains permanently its transmissibility for the laser beam because no significant pollution occurs on its surface by means of ejected particles. The particles cannot become baked onto the optical element. Thermal overload of the external optical element and the accompanying mechanical damage is reliably prevented. In a suitable configuration, the protective screen can have a high and constant optical transmission for the laser beam so that sufficient treatment energy is available at the target surface. The protective screen itself has a high thermal damage threshold. It can be manufactured from a material, for example, metal, that is capable of withstanding high laser power passing through it or impinging on it. The protective screen is self-cleaning. Particles that have collected in or on the protective screen are burned off continuously by the incoming laser light so that the constructively provided transmission of the protective screen is maintained permanently. Even in the case of certain applications in which a high optical transmission is not a primary concern, the construction-based limitation of the optical transmission as a result of the presence of the protective screen is maintained at a constant predictable level.

The protective screen is comprised of structural elements that delimit screen openings. In a preferred embodiment, these structural elements, at least at their surface, are electrically conducting and are connected to an electric potential, in particular, in the form of electric ground. In this way, the effect is utilized that the particles ejected by the laser beam are electrically charged or even ionized by interaction with the impinging laser beam. At least a significant portion of the ejected particles therefore is exposed by the electric potential of the protective screen to sufficiently high electrostatic attractive forces in order to be guided toward the structural elements. They impact on the structural elements and are lodged thereon until they are burnt off by the introduced laser beam. In this way, the external optical element can be shielded even from such particles whose particle size is smaller than the width of the screen openings. A comparatively large-mesh protective screen can be used that has a high optical transmission but still a minimal particle or debris transmission.

It can be expedient to make only the surface of the structural elements of the protective screen to be electrically conducting. This can be realized, for example, by metallic vapour deposition on a ceramic screen structure. In an expedient embodiment, the structural elements of the protective screen are made from metal and, in particular, from metal wire. In addition to high electrical conductivity, this provides also high thermal load capacity. The wire can be easily made into the desired screen configuration as a welded or soldered arrangement or as a woven fabric.

The structural elements of the protective screen are advantageously in the form of, in particular, a square rectangular grid. In this way, a constructively predetermined screen width can be precisely and reproducibly adjusted with minimal expenditure.

The surface of the structural elements of the protective screen is preferably optically highly reflective. This can be realized, for example, by uncoated, metallic bright noncorrosive metal surfaces, for example, made from stainless steel or particularly by means of metallizing the surface. In this way, the thermal load of the protective screen as a result of laser beams impinging on the structural elements is reduced.

The protective screen has a spacing relative to the external optical element. Advantageously, this spacing is adjustable. For certain applications, it can be expedient to guide the laser system in such a way to the target surface or the treatment surface that it is not within the focus of the optical delivery system. In this connection, the protective screen generates on the target surface a grid-like dot pattern of the laser beam. By changing the spacing between the protective screen and the external optical element, the size and number of effective beam dots can be changed or matched to the application, respectively.

The laser system comprises spacer means for maintaining the spacing of the protective screen relative to the target surface. By means of the spacer means, depending on the application, the spacing of the protective screen relative to the target surface can be adjusted to be smaller or greater than the focal length or even identical to the focal length of the optical delivery system. In this way, it is possible to vary the intensity of the individual grid-like laser dots on the target surface. In particular, the maximum intensity of individual laser dots can be increased up to a factor of two and even of three compared to a laser illumination without protective screen. When the focal point is positioned at least approximately in the target plane, the protective screen does not create a dot pattern of the laser beam on the target plane. Instead, the approximate same intensity distribution is generated on the target surface as in an arrangement without protective screen. The protective screen therefore functions only as a protection means in this situation. As a whole, several adjusting possibilities for different applications are available.

Embodiments of the invention will be explained in the following with the aid of the drawing in more detail. It is shown in:
- Fig. 1: in a schematic longitudinal section a laser system according to the prior art being used for surgical removal (ablation) of body tissue;
- Fig. 2: the laser system according to Fig. 1 where the external optical element is exposed to particles that are ejected by the laser beam;
- Fig. 3: an embodiment of the laser system according to Fig. 1 and 2 with a protective screen according to the invention;
- Fig. 4: a schematic plan view of the protective screen according to Fig. 3 with details of its geometric configuration;
- Fig. 5: a diagrammatic illustration of the optical transmission and debris transmission of the protective screen according to Figs. 3 and 4 as a function of its geometric parameters;
- Fig. 6: a schematic side view of the optical delivery system according to Figs. 3 to 5 with an illustration of the course of the beam in an arrangement where the target surface is in front of the focal point;
- Fig. 7: a diagrammatic illustration of the curve of the irradiance across the cross-section of the illuminated surface in the arrangement according to Fig. 6;
- Fig. 8: a diagrammatic illustration of the curve of the irradiance across the cross-section of the illuminated surface where the target surface is displaced toward the focal point as compared to the arrangement according to Fig. 6;
- Fig. 9: a variant of the arrangement according to Fig. 6 with an illustration of the course of the beam in an arrangement where the target surface is at the focal point;
- Fig. 10: a further variant of the arrangement according to Fig. 6 and Fig. 9 with an illustration of the course of the beam where the target surface is arranged behind the focal point;
- Fig. 11: a diagrammatic illustration of the course of the irradiance across the cross-section of the illuminated surface in an arrangement according to Fig. 10.

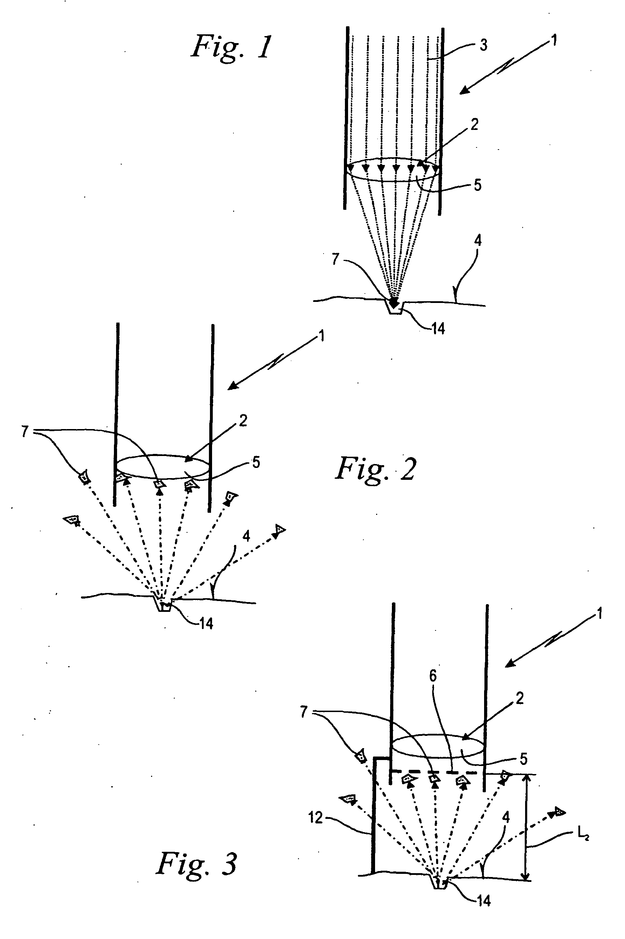

Fig. 1 shows in a schematic longitudinal illustration a laser system 1 according to the prior art during surgical removal of body tissue. The laser system 1 comprises an optical delivery system 2 of which, for simplification of the drawing, only an external optical element 5 in the form of a glass lens that is facing a target surface 4 is illustrated. In operation of the laser system 1 a laser beam 3 is guided by means of the optical delivery system 2 toward a target surface 4 or a treatment surface. In the application according to Fig. 1, particles 7 are excised from the target surface 4 so that a depression 14 is produced.

Fig. 2 shows the laser system according to Fig. 1 after excision of the particles 7. The energy of the laser beam 3 generates heat at the target surface 4 that leads to sudden evaporation of water and other liquids. In this way, microexplosions are generated that excise the particles 7 and eject them. In the illustration according to Fig. 2 it can be seen that the particles 7 reach the external surface of the external optical element 5. They can damage or soil the optical element 5. Even if no direct damage of the external optical element 5 is caused by the ejected particles, an indirect damage can still occur in that the particles 7 will lodge on the external optical element and, as a result of the impinging laser energy, cause thermal overload and damage to the external optical element 5.

Fig. 3 shows an embodiment according to the invention for improving the laser system 1 according to Figs. 1 and 2. The inventive laser system 1 is provided for medical removal (ablation) of body tissue as indicated by the target surface 4. However, other types of medical applications or cosmetic applications without removal of body tissue can be envisioned. In all applications, it can occur that individual particles 7 are ejected from the target surface 4. For protecting the external optical element 5 of the optical delivery system 2, a mechanical filter in the form of a protective screen 6 is therefore provided at the exit side that is facing toward the target surface 4. At least most of the ejected particles 7 that move toward the external optical element 5 are trapped by the protective screen 6 so that theses particles cannot reach the external surface of the optical element 5.

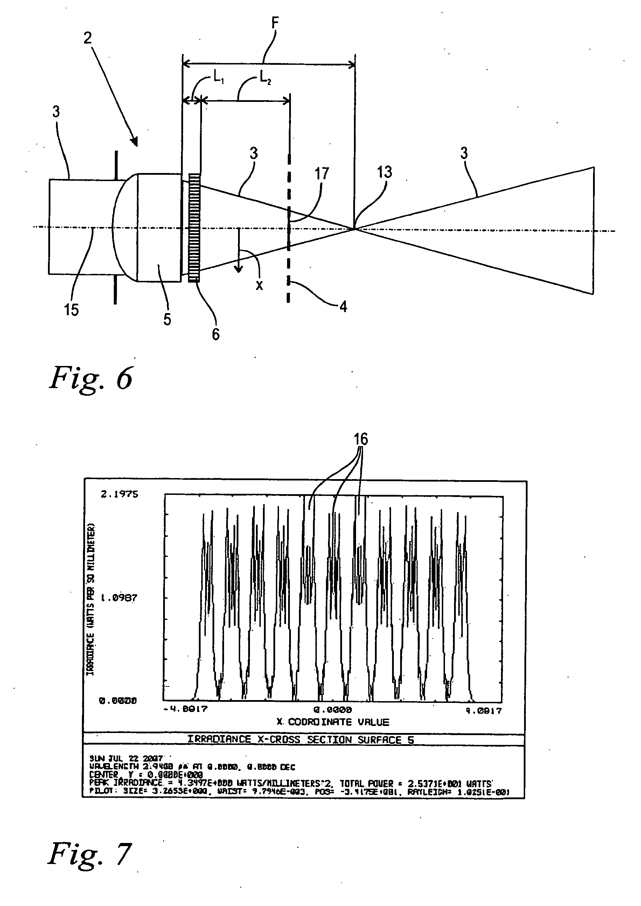

The illustration according to Fig. 3 also shows that the inventive laser system 1 is provided with schematically indicated spacer means 12 for maintaining the spacing L₂ of the protective screen 6 relative to the target surface 4. Further details in regard to the function of the spacer means 12 will be explained in more detail in connection with Figs. 6 to 11.

In the illustrated embodiment, the external optical element 5 is configured as a glass lens that is illustrated schematically. However, a protective glass plate or any other type of optical element 5 that is transmissive or reflective for the laser beam 3 can be provided instead.

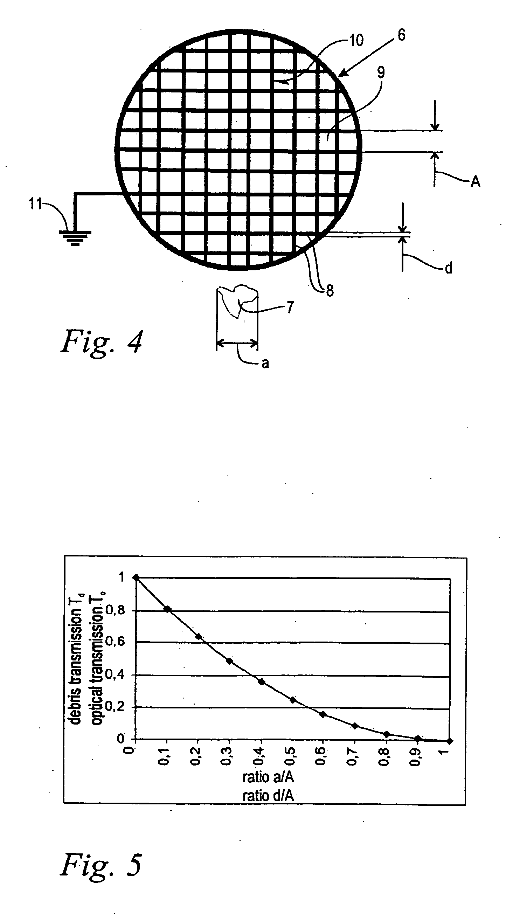

Fig. 4 shows a schematic plan view of the protective screen 6 according to Fig. 3 for providing the mechanical filter according to the invention. The protective screen 6 is comprised of structural elements 8 that delimit between them the screen openings 9. In this connection, the term mechanical filter is to be understood in that the structural elements 8 prevent the passage of at least most of the ejected particles 7 while the laser beam 3 (Fig. 3) can pass the screen openings 9 unhindered. The screen openings 9 are completely free, i.e., in operation they are filled with air, and are not filled with glass or any other mechanically opaque materials.

The protective screen 6 can be perforated sheet metal with for example punched screen openings 9, an eroded structure or a laser-cut structure. In the illustrated embodiment, the structural elements 8 of the protective screen 6 are made from metal and woven or knitted from metal wire. The wire is bright so that the surface 10 of the structural elements 8 as well as the interior of the wire cross-section is electrically conducting. It can also be expedient to provide a non-conducting support body for forming the structural elements 8, for example, made from ceramic material, wherein the surface 10 is then to be coated so as to be electrically conducting. At least the electrically conducting surface 10 and in this case the entire electrically conducting structural elements 8 are connected in an electrically conducting way to electric potential. The electric potential in the illustrated embodiment is electric ground 11. However, a type of electric potential different from electric ground 11 can be expedient.

In the illustrated embodiment, the structural elements 8 of the protective screen 6 made from wire material are arranged in the form of a square rectangular grid. A different arrangement of the structural elements 8 can also be expedient wherein the screen openings 9 are in the form of elongate rectangles or of a deviating shape, optionally of a circular or an irregular shape. The screen openings 9 have an average width A while the ejected particles 7 have an average particle size a. The width A of the screen openings 9 is of the same magnitude as the average particle size a. It can be minimally larger and is preferably at least a little smaller than the average particle size a.

Fig. 5 shows in a theoretically approximated form a diagrammatic illustration of the debris transmission T_{d} of the protective screen 6 (Fig. 4) for the particles 7 as a function of the ratio of the average particle size a to the width A of the screen openings 9. The ratio of the average particle size a to the width A is expediently at least 0.2; in the illustrated embodiment, it is at least 0.4. In this way, a theoretically approximated debris transmission T_{d} of less than 0.35 is achieved. This means that, in first approximation and without further measures, only 35 % of all particles 7 (Fig. 4) or less can pass the screen openings 9.

The remaining larger portion of the particles 7 is retained by the protective screen 6. Since as an additional measure, in accordance with the illustration of Fig. 4, an electric ground is provided for the protective screen 6, even those particles 7 that have a significantly smaller size and, based only on their size, could pass the screen openings 9, are attracted and trapped by the protective screen 6. In this way, the practical actual transmission T_{d} is significantly below the above-mentioned values taken from the diagram of Fig. 5. As a whole, the actual proportion of particles 7 passing through the protective screen 6 is negligibly small.

The diagrammatic illustration according to Fig. 5 shows also in theoretically approximated form in combination with Fig. 4 the optical transmission Tₒ of the protective screen 6, i.e., its optical transmission for the laser beam 3 (Fig. 3) as a function of the ratio of structural width d of the structural elements 8 relative to the width A of the screen openings 9. The ratio of structural width d to the width A of the screen openings is expediently < 0.5, preferably < 0.3, and in particular ≤ 0.1. For a ratio of 0.1 the optical transmission Tₒ is approximately 0.8 so that approximately 80% of the incoming laser energy can pass through the protective screen 6. The energy quantity is available for treatment of the target surface 4 (Fig. 3) while the energy quantity that is trapped at the structural width d of the structural elements 8 serves for burning off the particles 7 that have lodged on the protective screen 6. For certain applications, a very high optical transmission Tₒ is not important. A greater structural width d relative to the width A can be utilized. In accordance with the illustration of Fig. 5, the optical transmission Tₒ will drop. For a ratio of the structural width d to width A of 0.3 optical transmission is still approximately 0.5, i.e., 50 %, and for a ratio of 0.5 it is still approximately 0.25, i.e., 25 %. The difference relative to one or 100 % is trapped on the structural elements 8 and partially converted to heat. In order to prevent overheating of the protective screen 6, the surface 10 of the structural elements 8 is optically highly reflective. This can be realized, for example, by a noncorrosive metallized surface of the structural elements 8 or, for example, by employing stainless steel wire. In this way, a significant portion of the laser energy impinging on the structural elements 8 is reflected. Only the portion that is not reflected contributes to heating of the protective screen 6 or to burning off particles 7, without the protective screen 6 itself being damaged.

Fig. 6 shows a schematic side view of the optical delivery system 1 according to Figs. 3 to 5 with an illustration of the course of the laser beam 3. The optical delivery system 2 extends, like the laser beam 3 passing through it, along a longitudinal axis 15. The optical delivery system 2 and also the laser beam 3 are configured to have rotational symmetry relative to the longitudinal axis 15. The external optical element 5 is configured, for example, as a plane convex glass lens whose plane side is facing toward the target surface 4. The protective screen 6 is positioned at the exit side of the optical element 5 at a minimal spacing L₁ thereto. The spacing L₁ of the protective screen 6 relative to the external optical element 5 is adjustable.

The approximately cylindrically extending laser beam 3 that is distributed uniformly across its cross-section enters axis-parallel to the longitudinal axis 15 into the optical delivery system 2 and is focused therein. The optical delivery system 2 has a focal length F so that, at the spacing F relative to the optical delivery system 2, a focus 13 is generated. The laser beam 3 is focused in the optical delivery system 2 in such a way that its cross-section, beginning at the optical delivery system 2, tapers continuously until it reaches at least approximately a point focus at the focus 13. At the exit side of the focus 13 the cross-section of the laser beam 3 increases again continuously.

In the embodiment according to Fig. 6, the spacer means 12 illustrated in Fig. 3 are adjusted such that the protective screen 6 is positioned at a spacing L₂ relative to the target surface 4. The adjusted spacing L₂ in the embodiment according to Fig. 6 is smaller than the focal length F; the target surface 4 is thus positioned between the protective screen 6 and the focus 13. On the target surface 4, a surface area 17 is illuminated that is delimited by the circumferential contour of the laser beam 3.

Fig. 7 shows a diagrammatic illustration of the curve of irradiance across the cross-section of the surface area 17 of the target surface 4 illuminated according to Fig. 6 by laser beam 3. As one of the two cross-sectional coordinates, the direction X illustrated in Fig. 6 and beginning at the longitudinal axis 15 is shown. The spacing L₁ of the protective screen 6 from the external optical element 5 is sufficiently small so that the laser beam 3 passes through the maximum number of screen openings 9 (Fig. 4). In the illustrated embodiment according to Figs. 6 and 7, there are a total of eleven screen openings 9 (Fig. 4) in the X direction as can be seen in the intensity curve in the direction X shown in Fig. 7. Because the target surface 4 is not within the focus 13, one approximately point-shaped peak 16 results for each screen opening 9 (Fig. 4). These peaks 16 in the X direction are distributed from approximately -4 mm to approximately +4 mm so that the illuminated surface area 17 has a diameter of approximately 8 mm.

The ratio of by the spacer means 12 (Fig. 3) adjusted spacing L₂ to the focal width F is advantageously in a range from 0.1, inclusive, to 0.8, inclusive. In the configuration according to Fig. 6 and Fig. 7, the spacing L₂ is approximately 5.1 mm while the focal length F is 40.2 mm. The ratio of both relative to one another is thus approximately 0.13. The diagram according to Fig. 7 shows in this connection that the individual peaks 16 have a relatively minimal maximum value; however, they exhibit a broad full curve. All peaks 16 have approximately the same maximum value.

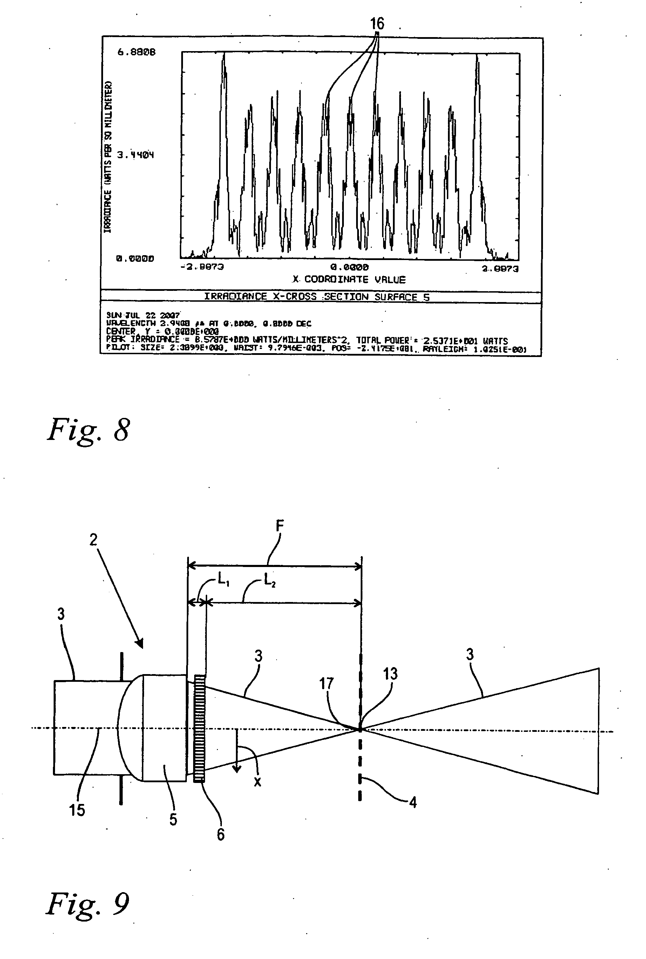

Fig. 8 shows a diagrammatic illustration of the curve of irradiance for an arrangement according to Fig. 6 wherein, however, by means of the spacer means 12 (Fig. 3) the spacing L₂ has been enlarged. The target surface 4 (Fig. 6) is still between the protective screen 6 and the focus 13 but, in comparison to the illustration of Fig. 7, is moved toward the focus 13, i.e., is closer to the focus 13. Accordingly, the illuminated surface area 17 is smaller and, in accordance with the illustration of Fig. 8, this leads to higher maximum values of the peaks 16. In average, the values of the peaks 16 are approximately two times higher compared to a value without the protective screen 6. When looking at Figs. 6 and 8, values for the X direction of the illuminated surface area 17 of approximately -3 mm up to approximately +3 mm result so that the illuminated surface area 17 has a diameter of approximately 6 mm. In the border area, i.e., for X values having a high amount, the maximum values of the peaks 16 are greater than in the central area for X values near zero.

The spacing L₂ according to Fig. 8 is approximately 15.1 mm for an unchanged focal length F of 40.2 mm. Their relative ratio is therefore approximately 0.38 mm.

Fig. 9 shows a variant of the arrangement according to Fig. 6 with an illustration of the course of the laser beam 3 wherein the spacing L₂ is adjusted such that the focus 13 is located at least approximately on the target surface 4. The illuminated surface area 17 is very small but has an at least approximately uniform illumination or intensity distribution of the laser energy. Peaks 16, as they are illustrated in Figs. 7 and 8, do not occur. However, the protective function of the protective screen 6 against ejected particles 7 (Fig. 3) remains intact.

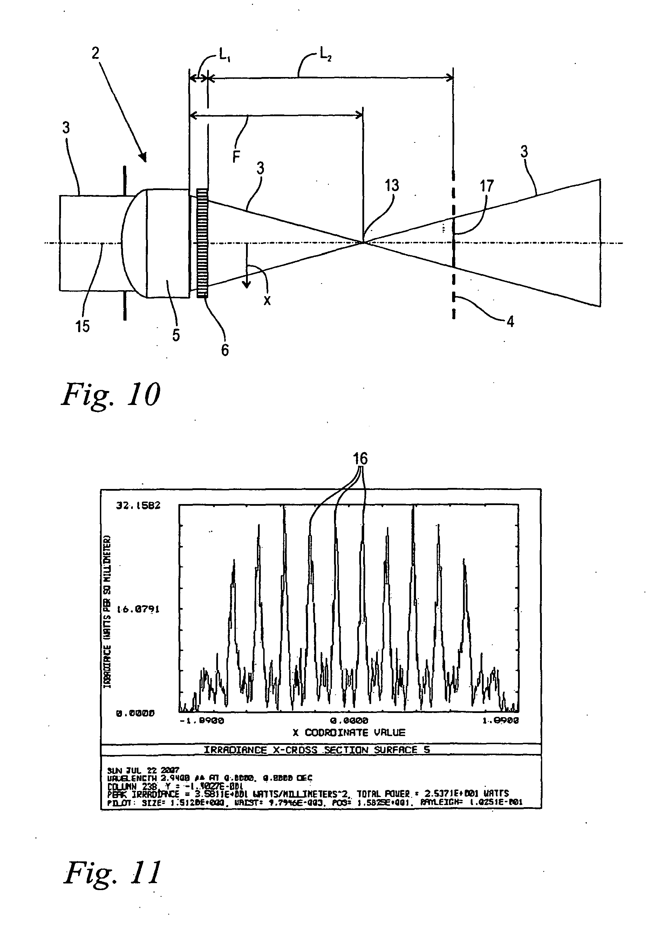

Fig. 10 shows a further variant of the arrangement according to Figs. 6 and 9 wherein the adjusted spacing L₂ is greater than the focal length F. Relative to the optical delivery system 2 with the protective screen 6, the target surface 4 is thus on the opposite side of the focus 13. The adjusted spacing L₂ according to Fig. 10 is 55.1 mm with unchanged focal length of 40.2 mm. The ratio of both is preferably in a range of 1.2, inclusive, to 3.0, inclusive, and is approximately 1.37 in accordance with Fig. 10.

In analogy to the illustrations of Figs. 7 and 8, the irradiance curve at the illuminated surface area 17 is illustrated in Fig. 11. The diameter of the illuminated surface area 17 is approximately 4 mm. In comparison to Figs. 7 and 8, the curve of the individual peaks 16 is more pointed with higher maximum values. Moreover, the maximum values in the center area are higher than in the edge area. In average, the values of the peaks 16 are approximately three times higher compared to a value without the protective screen 6.

As a whole, by changing or adjusting the spacings L₁ and L₂ different illumination patterns with different illumination intensities according to Figs. 6 to 11 can be adjusted. For ablative applications, a uniform illumination profile can be expedient as is achieved in accordance with Fig. 9 with the protective function of the protective screen 6. For other applications such as making hardened burn scars more flexible or similar applications, a grid-shaped or point-shaped illumination pattern according to Figs. 6 to 8, 10 and 11 can be expedient. Such pattern is generated by the protective screen 6 while utilizing its protective function and adjusted, as needed, by changing the spacings L₁ and L₂ as appropriate for the respective application.

## Claims

1. A laser system (1) for medical and cosmetic applications, comprising an optical delivery system (2) for guiding a laser beam (3) to a target surface (4) wherein the optical delivery system (2) has an external optical element (5) facing toward the target surface (4), further comprising a mechanical filter for shielding the external optical element (5) from particles (7) ejected away from the target surface (4) by the laser beam (3) arranged at the exit side of the external optical element (5),
**characterized in that** the mechanical filter is in the form of a protective screen (6), wherein said protective screen (6) is comprised of structural elements (8) that delimit screen openings (9), and wherein the laser system (1) has spacer means (12) for maintaining a spacing (L₂) of the protective screen (6) relative to the target surface (4).

2. The laser system according to claim 1,
**characterized in that** the structural elements (8), at least at their surface (10), are electrically conducting and are connectable to electric potential, in particular, in the form of electric ground (11).

3. The laser system according to claim 1 or 2,
**characterized in that** the structural elements (8) of the protective screen (6) are comprised of metal and, in particular, of a metal wire.

4. The laser system according to one of the claims 1 to 3,
**characterized in that** the structural elements (8) of the protective screen (6) are arranged in the shape of a rectangular grid that is, in particular, a square rectangular grid.

5. The laser system according to one of the claims 1 to 4,
**characterized in that** the surface (10) of the structural elements (8) of the protective screen (6) is configured to be optically highly reflective.

6. The laser system according to one of the claims 1 to 5,
**characterized in that** the protective screen (6) has a spacing (L₁) relative to the external optical element (5) wherein the spacing (L₁) is adjustable.

7. The laser system according to one of the claims 1 to 6,
**characterized in that** the optical delivery system (2) has a focal length (F), **in that** the spacing (L₂) adjusted by the spacer means (12) is smaller than the focal length (F) and **in that**, in particular, the ratio of the spacing (L₂) to the focal length (F) is in a range from 0.1, inclusive, to 0.8, inclusive.

8. The laser system according to one of the claims 1 to 6,
**characterized in that** the optical delivery system (2) has a focal length (F), and **in that** the spacing (L₂) adjusted by means of the spacer means (12) corresponds at least approximately to the focal length (F).

9. The laser system according to one of the claims 1 to 6,
**characterized in that** the optical delivery system (2) has a focal length (F), **in that** the spacing (L₂) adjusted by means of the spacer means (12) is greater than the focal length (F) and **in that**, in particular, the ratio of the spacing (L₂) relative to the focal length (F) is within a range of 1.2, inclusive, to 3.0, inclusive.

10. The laser system according to one of the claims 1 to 9,
**characterized in that** the structural elements (8) of the protective screen (6) have a structural width (d), wherein the ratio of the structural width (d) to the width (A) of the screen openings is < 0.5, preferably < 0.3, and, in particular, ≤ 0.1.

## Patentansprüche

1. Lasersystem (1) für medizinische und kosmetische Anwendungen, umfassend ein optisches Liefersystem (2) zur Leitung eines Laserstrahls (3) zu einer Zielfläche (4), wobei das optische Liefersystem (2) ein äußeres, der Zielfläche (4) zugewandtes optisches Element (5) aufweist, umfassend des weiteren einen mechanischen Filter zur Abschirmung des äußeren optischen Elementes (5) gegen vom Laserstrahl (3) aus der Zielfläche (4) ausgelöste Partikel (7), welcher ausgangsseitig des äußeren optischen Elementes (5) angeordnet ist,
**dadurch gekennzeichnet, dass** der mechanische Filter in Form eines Schutzgitters (6) ausgeführt ist, wobei das Schutzgitter (6) durch Strukturelemente (8) gebildet ist, die Gitteröffnungen (9) begrenzen, und wobei das Lasersystem (1) Abstandsmittel (12) zur Einhaltung eines Abstandes (L₂) des Schutzgitters (6) gegenüber der Zielfläche (4) aufweist.

2. Lasersystem nach Anspruch 1,
**dadurch gekennzeichnet, dass** die Strukturelemente (8) zumindest an ihrer Oberfläche (10) elektrisch leitend und mit einem elektrischen Potenzial insbesondere in Form einer elektrischen Erde (11) verbindbar sind.

3. Lasersystem nach Anspruch 1 oder 2,
**dadurch gekennzeichnet, dass** die Strukturelemente (8) des Schutzgitters (6) aus Metall und insbesondere aus metallischem Draht sind.

4. Lasersystem nach einem der Ansprüche 1 bis 3,
**dadurch gekennzeichnet, dass** die Strukturelemente (8) des Schutzgitters (6) in Form eines insbesondere quadratischen Rechteckgitters angeordnet sind.

5. Lasersystem nach einem der Ansprüche 1 bis 4,
**dadurch gekennzeichnet, dass** die Oberfläche (10) der Strukturelemente (8) des Schutzgitters (6) optisch hoch reflektierend ausgebildet ist.

6. Lasersystem nach einem der Ansprüche 1 bis 5,
**dadurch gekennzeichnet, dass** das Schutzgitter (6) einen Abstand (L₁) zum äußeren optischen Element (5) aufweist, wobei der Abstand (L₁) einstellbar ist.

7. Lasersystem nach einem der Ansprüche 1 bis 6,
**dadurch gekennzeichnet, dass** das optische Liefersystem (2) eine Brennweite (F) aufweist, dass der durch die Abstandsmittel (12) eingestellte Abstand (L₂) kleiner als die Brennweite (F) ist, und dass insbesondere das Verhältnis des Abstandes (L₂) zur Brennweite (F) in einem Bereich von einschließlich 0,1 bis einschließlich 0,8 liegt.

8. Lasersystem nach einem der Ansprüche 1 bis 6,
**dadurch gekennzeichnet, dass** das optische Liefersystem (2) eine Brennweite (F) aufweist, und dass der durch die Abstandsmittel (12) eingestellte Abstand (L₂) zumindest näherungsweise der Brennweite (F) entspricht.

9. Lasersystem nach einem der Ansprüche 1 bis 6,
**dadurch gekennzeichnet, dass** das optische Liefersystem (2) eine Brennweite (F) aufweist, dass der durch die Abstandsmittel (12) eingestellte Abstand (L₂) größer als die Brennweite (F) ist, und dass insbesondere das Verhältnis des Abstandes (L₂) zur Brennweite (F) in einem Bereich von einschließlich 1,2 bis einschließlich 3,0 liegt.

10. Lasersystem nach einem der Ansprüche 1 bis 9,
**dadurch gekennzeichnet, dass** die Strukturelemente (8) des Schutzgitters (6) eine Strukturbreite (d) aufweisen, wobei das Verhältnis der Strukturbreite (d) zur Weite (A) der Gitteröffnungen < 0,5, bevorzugt < 0,3 und insbesondere ≤ 0,1 ist.

## Revendications

1. Système laser (1) pour applications médicales et cosmétiques, comprenant un système de distribution optique (2) pour guider un faisceau laser (3) vers une surface cible (4), dans lequel le système de distribution optique (2) comporte un élément optique externe (5) faisant face à la surface cible (4), comprenant en outre un filtre mécanique pour protéger l'élément optique externe (5) contre les particules (7) éjectées de la surface cible (4) par le faisceau laser (3) agencé sur le côté de sortie de l'élément optique externe (5),
**caractérisé en ce que** le filtre mécanique est sous la forme d'un écran de protection (6), dans lequel ledit écran de protection (6) se compose d'éléments structurels (8) qui délimitent des ouvertures d'écran (9), et dans lequel le système laser (1) comporte un moyen d'entretoise (12) pour maintenir un espacement (L₂) de l'écran de protection (6) par rapport à la surface cible (4).

2. Système laser selon la revendication 1,
**caractérisé en ce que** les éléments structurels (8), au moins au niveau de leur surface (10), sont électriquement conducteurs et peuvent être connectés à un potentiel électrique, en particulier, sous la forme d'une masse électrique (11).

3. Système laser selon la revendication 1 ou 2,
**caractérisé en ce que** les éléments structurels (8) de l'écran de protection (6) se composent de métal et, en particulier, d'un fil métallique.

4. Système laser selon l'une des revendications 1 à 3,
**caractérisé en ce que** les éléments structurels (8) de l'écran de protection (6) sont agencés sous la forme d'une grille rectangulaire qui est, en particulier, une grille rectangulaire carrée.

5. Système laser selon l'une des revendications 1 à 4,
**caractérisé en ce que** la surface (10) des éléments structurels (8) de l'écran de protection (6) est configurée pour être optiquement très réfléchissante.

6. Système laser selon l'une des revendications 1 à 5,
**caractérisé en ce que** l'écran de protection (6) a un espacement (L₁) par rapport à l'élément optique externe (5), dans lequel l'espacement (L₁) est ajustable.

7. Système laser selon l'une des revendications 1 à 6,
**caractérisé en ce que** le système de distribution optique (2) a une longueur focale (F), **en ce que** l'espacement (L₂) ajusté par le moyen d'entretoise (12) est plus petit que la longueur focale (F) et **en ce que**, en particulier, le rapport de l'espacement (L₂) sur la longueur focale (F) est dans une plage de 0,1 inclus à 0,8 inclus.

8. Système laser selon l'une des revendications 1 à 6,
**caractérisé en ce que** le système de distribution optique (2) a une longueur focale (F), et **en ce que** l'espacement (L₂) ajusté par le biais du moyen d'entretoise (12) correspond au moins approximativement à la longueur focale (F).

9. Système laser selon l'une des revendications 1 à 6,
**caractérisé en ce que** le système de distribution optique (2) a une longueur focale (F), **en ce que** l'espacement (L₂) ajusté par le biais du moyen d'entretoise (12) est plus grand que la longueur focale (F) et **en ce que**, en particulier, le rapport de l'espacement (L₂) sur la longueur focale (F) est dans une plage de 1,2 inclus à 3,0 inclus.

10. Système laser selon l'une des revendications 1 à 9,
**caractérisé en ce que** les éléments structurels (8) de l'écran de protection (6) ont une largeur structurelle (d), dans lequel le rapport de la largeur structurelle (d) sur la largeur (A) des ouvertures d'écran est < 0,5, de préférence < 0,3, et en particulier ≤ 0,1.
